(19) Europäisches Patentamt
European Patent Office
Office européen des brevets

(11) EP 1 682 192 B1

(12) # EUROPÄISCHE PATENTSCHRIFT

(45) Veröffentlichungstag und Bekanntmachung des Hinweises auf die Patenterteilung:
**07.03.2012 Patentblatt 2012/10**

(21) Anmeldenummer: **04790950.2**

(22) Anmeldetag: **28.10.2004**

(51) Int Cl.:
***A61L 15/48** (2006.01)*

(86) Internationale Anmeldenummer:
**PCT/EP2004/012177**

(87) Internationale Veröffentlichungsnummer:
**WO 2005/042039 (12.05.2005 Gazette 2005/19)**

(54) **BLUT UND/ODER KÖRPERFLÜSSIGKEITEN ABSORBIERENDES HYDROGEL**

BLOOD- AND/OR BODY FLUID-ABSORBING HYDROGEL

HYDROGEL ABSORBANT LE SANG ET/OU DES LIQUIDES ORGANIQUES

(84) Benannte Vertragsstaaten:
**AT BE BG CH CY CZ DE DK EE ES FI FR GB GR HU IE IT LI LU MC NL PL PT RO SE SI SK TR**

(30) Priorität: **31.10.2003 DE 10351267**
**28.06.2004 DE 102004035671**

(43) Veröffentlichungstag der Anmeldung:
**26.07.2006 Patentblatt 2006/30**

(73) Patentinhaber: **BASF SE**
**67056 Ludwigshafen (DE)**

(72) Erfinder:
- **BECK, Martin**
**67133 Maxdorf (DE)**
- **FRENZ, Volker**
**67317 Altleiningen (DE)**
- **KOWALSKI, Anna**
**67227 Frankenthal (DE)**
- **SELZER, Elisabeth**
**67122 Altrip (DE)**
- **BAUER, Ernst, Jürgen**
**67069 Ludwigshafen (DE)**
- **KELLER, Harald**
**67069 Ludwigshafen (DE)**
- **STEINMETZ, Bernhard**
**97535 Rütschenhausen (DE)**

(56) Entgegenhaltungen:
**EP-A- 0 705 643**
**EP-A- 1 072 630**
**WO-A-95/17455**
**US-A- 5 847 031**
**US-A1- 2002 128 618**
**US-B1- 6 592 768**

- **PATENT ABSTRACTS OF JAPAN Bd. 2000, Nr. 19, 5. Juni 2001 (2001-06-05) & JP 2001 046423 A (SHISEIDO CO LTD), 20. Februar 2001 (2001-02-20)**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Blut und/oder Körperflüssigkeiten absorbierende Hydrogele, ein Verfahren zur Herstellung der Blut und/oder Körperflüssigkeiten absorbierenden Hydrogele sowie deren Verwendung.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erindung zu verlassen.

Quellbare hydrogelbildende Polymere, sogenannte Superabsorber (Super-Absorbing Polymers, SAP), sind aus dem Stand der Technik bekannt.

Quellbare hydrogelbildende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Hydrogele werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau oder zur Verdickung aller Arten von Abfällen, insbesondere von medizinischen Abfällen, verwendet.

Quellbare hydrogelbildende Polymere sind bevorzugt solche mit einer Absorption von 0,9 Gew.-%iger Kochsalzlösung von mindestens dem 10-fachen Eigengewicht bezogen auf das eingesetzte Polymer, bevorzugt dem 20-fachen Eigengewicht. Diese Absorption wird bevorzugt auch unter einem Druck beispielsweise von 4,83 kPa (0,7 psi) erreicht.

Zur Verbesserung der Anwendungseigenschaften werden quellbare hydrogelbildende Polymere üblicherweise oberflächen- oder gelnachvernetzt.

Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Abfälle, insbesondere medizinische Abfälle und jede Art von Abfällen, die mit giftigen, ansteckenden oder für Mensch und Umwelt gefährlichen Stoffen belastet sind, müssen sicher gehandhabt und transportiert werden. Ein Superabsorber kann durch die Aufnahme des flüssigen Abfalls die meisten gefährlichen Stoffe immobilisieren.

Medizinische Abfälle, besonders Krankenhausabfälle aus Operationsräumen, bestehen vor allem aus Blut, Körperflüssigkeiten und physiologischer Kochsalzlösung, die als Spüllösung Verwendung findet.

Herkömmliche Superabsorber sind daraufhin optimiert, in Hygieneartikeln, vor allem Babywindeln, Urin zu absorbieren. Sie zeigen immer eine wesentlich geringere Absorption von Blut im Vergleich zu synthetischem Urin oder physiologischer Kochsalzlösung. Unterscheidet sich die Absorption von Blut und physiologischer Kochsalzlösung wesentlich, so müssen je nach Zusammensetzung des Abfalls unterschiedliche Mengen Superabsorber zugegeben werden. Dies würde in der Praxis bedeuten, dass Superabsorber entweder grundsätzlich weit überdosiert zugegeben werden muss, oder dass gegebenenfalls Superabsorber nachdosiert werden muss. Dabei müsste der eigentlich verschlossene Behälter allerdings gegebenenfalls mehrfach wieder geöffnet werden, welches zusätzliche Zeit benötigt und ein Risiko für Personen darstellt, die mit den Abfällen umgehen müssen.

Es ist daher äußerst vorteilhaft einen Superabsorber bereitzustellen, der in der Lage ist, größere Mengen Blut als handelsübliche Superabsorber zu absorbieren. Bei Krankenhausabfällen und medizinischen Abfällen können somit Lösungen mit unterschiedlichen Mengen an Blut gleichgut verfestigt werden.

Ein weiterer Aspekt bei der Behandlung medizinischer Abfälle mit Superabsorbern ist die Absorptionsgeschwindigkeit. Bei handelsüblichen Superabsorbern, besonders für Babywindeln und Inkontinenzprodukte, ist eine sehr hohe Absorptionsgeschwindigkeit nicht vorteilhaft, da sich die zu absorbierende Flüssigkeit zunächst über den absorbierenden Artikel verteilen soll. Ein Superabsorber mit sehr hoher Quellgeschwindigkeit erlaubt nicht die gleichmäßige Verteilung des Flüssigkeit in dem Hygieneartikel sondern absorbiert die gesammte Flüssigkeit sofort. Im Gegensatz zu herkömmlichen Windeln ist bei medizinischen Abfällen und Produkten der Damenhygiene, wie Tampons, Binden und Einlagen, die rasche Absorption von Blut und/oder Körperflüssigkeiten entscheidend. Die hohe Absorptionsgeschwindigkeit ist wichtig, um die medizinischen Abfälle möglichst rasch zu absorbieren und damit zu immobilisieren.

Die rasche Verfestigung erlaubt eine schnellere und sicherere Handhabung dieser Abfälle, beispielsweise bei Transport und Lagerung.

Ein weiterhin vorteilhaftes Verhalten für die Absorption von medizinischen Abfällen ist es, wenn ein Teil des zugegebenen Superabsorbers nach der Zugabe zur Lösung auf der Flüssigkeitsoberfläche schwimmt, und ein weiterer Teil absinkt. Dadurch wird zum einen eine schnelle Verdickung erzeugt, da der Superabsorber von beiden Grenzflächen aus quellen kann, zum anderen wird die Oberfläche der Flüssigkeit sofort mit Superabsorber bedeckt und ein Austreten von Stoffen, wie beispielsweise Viren oder Bakterien, erschwert, insbesondere wird ein Verspritzen von Flüssigkeit vermieden.

Bei Produkten der Damenhygiene ist eine rasche Absorption wünschenswert, um die Flüssigkeit rasch vom

Körper weg zu transportieren und in dem Hygieneartikel zu speichern. Neben der raschen Absorption und hohen Quellgeschwindigkeit ist auch eine hohe Retention notwendig.

Für Hygieneartikel ist die höhere Blutabsorption ein wesentlicher Vorteil, da dadurch die Entwicklung von sehr effektiven und dünnen Hygieneartikeln möglich ist, die vom Kunden aufgrund des Tragekomforts bevorzugt werden.

Die Patentanmeldung WO-A-99/55767 beschreibt die Verwendung von Aluminaten zur Oberflächennachvemetzung von unvernetzten oder kovalent vernetzten Hydrogelen. Die Anmeldung lehrt, dass durch die nachträgliche Vernetzung die Gelstärke, die Absorption von Flüssigkeiten und Blut, insbesondere die Absorption unter Druck, verbessert wird. Die Anquellgeschwindigkeit und das Schwimmverhalten des nachvemetzten Hydrogels ist aber unbefriedigend.

Die Patentanmeldung DE-A-199 09 653 lehrt das Aufbringen einer wässrigen Lösung eines Kations vor oder nach einer Nachvemetzungsreaktion. Unabhängig davon ob diese Behandlung vor oder nach der Nachvemetzungsreaktion durchführt wird, können durch diese Behandlung die geforderten Eigenschaften nicht erreicht werden.

Die Patentanmeldung WO-A-00/10496 beschreibt ein optimiertes Material zur Absorption von Blut durch Aufbringen von Kaolinit auf befeuchteten Superabsorber und anschließende Trocknung. Die Anmeldung lehrt nicht die Kombination von verschiedenen Beschichtungen und ist allein nicht ausreichend, um Superabsorber mit den gewünschten Eigenschaften zu erhalten.

Die Patentanmeldung WO-A-95/17455 beschreibt einen porösen Superabsorber, der auch in gequollenen Zustand auf Wasser schwimmen kann. Dies wird durch dispergierten Stickstoff im Superabsorber erzeugt und kann nicht durch eine Nachbehandlung realisiert werden. Auch ist es anwendungstechnisch nicht von Vorteil, wenn der Superabsorber vollständig auf der Flüssigkeit schwimmt.

Die Patentanmeldung EP-A-0 759 460 beschreibt ein Material, welches durch Zusatz von großen Mengen eines Oberflächennachvernetzungsreagenzes erneut nachvernetzt wurde. Die höhere Vernetzung führt allerdings nicht zu Produkten mit sehr hoher Blutabsorption.

Die Patentanmeldung WO-A-95/19191 beschreibt die Herstellung von superabsorbierenden Materialien mit verbesserter Blutabsorption. Dazu werden handelsübliche Superabsorber zusätzlich mit Polyolen, wie beispielsweise Polyethylenglykol oder Glycerin besprüht. Die zusätzliche Vernetzung der Polymerstränge erfolgt dabei im wesentlichen durch Wasserstoffbrückenbindungen. Auch die nach der Lehre dieser Anmeldung erhältlichen Superabsorber eignen sich aufgrund fehlender Schwimmfähigkeit nicht zur Abdeckung von Flüssigkeitsoberflächen.

Die Patentanmeldungen WO-A-98/42193, CA-A-2,188,838, WO-A-95/15771 und JP-A-03/044367 beschreiben die Verwendung von Superabsorbern mit Bioziden, wie beispielsweise Polyvinylpyrrolidon/Jod-Komplexen und Glutardialdehyd. Dabei werden handelsübliche Superabsorber mit einem Biozid gemischt. Dies führt weder zu einer Verbesserung der Blutabsorption, noch zu einer Erhöhung der Absorptionsgeschwindigkeit, sondern allein zu einer Desinfektion der verdickten Lösung.

Die Patentanmeldung JP-A-06/345980 beschreibt die Mischung von Superabsorbern mit anionischen Tensiden. Dies verbessert weder die Blutabsorption, noch erhöht es die Absorptionsgeschwindigkeit.

Es bestand daher die Aufgabe, einen speziellen Superabsorber zu entwickeln, der optimal für die Verfestigung medizinischer Abfälle geeignet ist. Dieser Superabsorber muss durch schnelle Absorption sowie Verdickung oder Verfestigung eine sichere Handhabung von medizinischen Abfällen ermöglichen.

Eine weitere zu lösende Aufgabe besteht darin, einen Superabsorber zur Verfügung zu stellen, der gleichzeitig eine wesentlich erhöhte Blutabsorption und eine rasche Anquellgeschwindigkeit zeigt. Ein solcher Superabsorber wäre dann nicht nur für die Verdickung von Abfällen jeder Art, besonders medizinischer Abfälle geeignet, sondern könnte auch sehr gut zur Absorption von Flüssigkeiten bei Produkten der Damenhygiene, wie Tampons, Binden und Slipeinlagen, verwendet werden.

Eine weitere zu lösende Aufgabe besteht darin, einen Superabsorber zur Verfügung zu stellen, der nach Zugabe zur Lösung zumindest teilweise auf der Flüssigkeitsoberfläche schwimmt während ein weiterer Teil absinkt. Dadurch wird zum einen eine schnelle Verdickung flüssiger Abfälle bewirkt, da der Superabsorber von beiden Grenzflächen aus quellen kann, zum anderen wird die Oberfläche der Flüssigkeit sofort mit Superabsorber bedeckt und ein Austreten von Stoffen, wie beispielseise Viren, Bazillen oder Bakterien, erschwert.

Überraschend wurde nun gefunden, dass durch die erfindungsgemäße Nachbehandlung bekannter superabsorbierender Materialien mit hydrophoben und gegebenenfalls hydrophilen Verbindungen eine höhere Quellgeschwindigkeit und eine verbesserte Blutabsorption erreicht wird. Vorzugsweise werden partikuläre hydrophobe und gegebenenfalls hydrophile Verbindungen verwendet. Zur Nachbehandlung mit hydrophoben Verbindungen eignen sich insbesondere hydrophobierte pyrogene Kieselsäuren oder hydrophobierte Gemische aus pyrogenen Kieselsäuren und pyrogenen Aluminiumoxiden. Zur Nachbehandlung mit hydrophilen Verbindungen eignen sich insbesondere pyrogene Kieselsäuren oder Gemische aus pyrogenen Kieselsäuren und pyrogenen Aluminiumoxiden.

Bei den im erfindungsgemäßen Verfahren einsetzbaren superabsorbierenen Materialien handelt es sich um Hydrogele oder Hydrogelgemische. Der Wassergehalt der Hydrogele beträgt vorzugsweise weniger als 20 Gew.%, besonders bevorzugt weniger als 10 Gew.%, ganz besonders bevorzugt weniger als 1 Gew.-%.

Die Begriffe "hydrophob" und "hydrophil" beschreiben das Benetzungsverhalten einer Oberfläche mit Wasser.

Auf hydrophoben Oberflächen beträgt der Rand- bzw. Kontaktwinkel eines Wassertropfens <90° und auf hydrophilen Oberflächen beträgt der Rand- bzw. Kontaktwinkel eines Wassertropfens >90°. Der Rand- bzw. Kontaktwinkel wird beispielsweise in Colloid Polym. Sci., Band 259 (1981), Seiten 391 bis 394, beschrieben.

Beispielsweise befinden sich auf der Partikeloberfläche pyrogener Kieselsäuren freie Hydroxygruppen. Über diese Hydroxygruppen sind Wasserstoffbrückenbindungen möglich. Dadurch sind pyrogene Kieselsäuren hydrophil.

Durch Umsetzung pyrogener Kieselsäuren mit beispielsweise Trimethylchlorsilan können die freien Hydroxygruppen in Silylethergruppen umgewandelt werden. Die Silylethergruppen können keine Wasserstoffbrückenbindungen mehr ausbilden. Die pyrogene Kieselsäure wurde hydrophobiert.

Die Mengen an hydrophilen und hydrophoben Partikeln werden dabei vorteilhaft so gewählt, dass sowohl eine erhöhte Anquellgeschwindigkeit, als auch ein teilweises Schwimmen der Superabsorber-Partikel auf der Flüssigkeitsoberfläche zu Beginn des Quellvorgangs erreicht wird. Durch die zusätzliche Beschichtung mit hydrophoben Partikeln verbleibt ein Teil des Superabsorbers nach Zugabe der gesamten notwendigen Superabsorbermenge zu der zu verdikkenden Flüssigkeit an der Oberfläche des zu verdickenden Materials. Ein weiterer Teil des so behandelten Superabsorbers sinkt langsam in die zu verdickende Lösung ein, da Superabsorber auf der Basis von Polyacrylaten normalerweise eine höhere Dichte haben als die zu verdickenden Lösungen. Ohne die Behandlung würde ein handelsüblicher Superabsorber einfach nur direkt nach Zugabe in die Lösung sinken.

Die Einsatzmenge an hydrophoben Partikeln ist dabei individuell einzustellen, wobei der Anteil des nachbehandelten Superabsorbers, der auf der zu verdickenden Flüssigkeit schwimmt, mit steigendem Gehalt hydrophober Partikel steigt. Wird der Superabsorber beispielsweise auch mit hydrophilen Stoffen nachbehandelt, so ist die Menge an hydrophoben Partikeln gegebenenfalls zu erhöhen.

Durch die zunächst partiell an der Oberfläche verbleibende Menge Superabsorber beginnt der Superabsorber nun sowohl vom Boden des Gefäßes nach oben, als auch von der Oberfläche nach unten in die zu verdickende Lösung zu quellen. Damit ergeben sich gleichzeitig mehrere anwendungstechnische Vorteile:

- Zum einen wird die zu verdickende Lösung schneller verfestigt, da ein Superabsorber, der von beiden Seiten quillt, nur die halbe Quellstrecke zurücklegen muß.

- Zum anderen ist die Oberfläche der zu verdickenden Lösung praktisch sofort mit Superabsorber belegt Der Superabsorber wirkt dann wie ein Korken auf dem Gefäß und erlaubt sicheren Transport und sichere Entsorgung der Abfälle, da während des Transports des Behälters keine flüssigen, kontaminierten Bestandteile mehr austreten können.

Durch die Kombination hydrophiler und hydrophober Partikel können Superabsorber mit hoher Quellgeschwindigkeit hergestellt werden, die zumindest teilweise zu Beginn des Quellvorgangs auf Wasser und wässrigen Oberflächen schwimmen und den Inhalt eines Gefäßes mit medizinischen Abfällen schnell verfestigen können.

Gegenstand der Erfindung ist ein Verfahren zur Nachbehandlung von absorbierenden Hydrogelen umfassend die Schritte:

- Nachbehandlung mit wenigstens einer hydrophoben Verbindung, vorzugsweise hydrophobe und/oder hydrophobierte Tonmineralien, hydrophobierte Aluminiumoxide und/oder hydrophobierte Kieselsäuren, besonders bevorzugt hydrophobierte Aluminiumoxide und/oder hydrophobierte Kieselsäuren, wie beispielsweise Aerosil® R 812, Aerosil® R 974 oder Aerosil® R 8200 (Degussa Aktiengesellschaft, DE).

- gegebenenfalls Nachbehandlung mit wenigstens einer hydrophilen Verbindung, vorzugsweise hydrophilen Tonmineralien, Aluminiumoxide und/oder Kieselsäuren, besonders bevorzugt Aluminiumoxide und/oder Kieselsäuren, wie beispielsweise Aerosil® 200 (Degussa Aktiengesellschaft, DE).

Vorzugsweise werden die hydrophoben und hydrophilen Verbindungen als Partikel eingesetzt. Die mittlere Partikelgröße beträgt üblicherweise 0,001 bis 10 $\mu$m, vorzugsweise 0,002 bis 5 $\mu$m, besonders bevorzugt 0,005 bis 1 $\mu$m und ganz besonders bevorzugt 0,01 bis 0,1 $\mu$m. Die Meßmethode für die Teilchengrößenverteilung basiert auf der Analyse der Beugungsspektren nach Fraunhofer. Die Analysen werden standardmäßig mit einem Mastersizer S, einem Lasergerät der Firma Malvern, durchgeführt. Besonders bevorzugt sind pyrogene Aluminiumoxide, pyrogene Kieselsäuren oder Gemische davon. Ganz besonders bevorzugt sind Gemische pyrogener Kieselsäuren mit größer 0 bis 20 Gew.-% pyrogenem Aluminiumoxid sowie pyrogene Kieselsäuren. Die mittlere Primärteilchengröße beträgt vorzugsweise 5 bis 50 nm, besonders bevorzugt 10 bis 20 nm, und die spezifische Oberfläche beträgt vorzugsweise 10 bis 1000 $m^2$/g, besonders bevorzugt 80 bis 380 $m^2$/g. Eingesetzt werden typischerweise 0,005 bis 20 Gew.-%, vorzugsweise 0,05 bis 10 Gew.-%, besonders bevorzugt 0,1 bis 5 Gew.-%, ganz besonders bevorzugt unter 1 Gew.-%, hydrophobe, hydrophobierter bzw. hydrophile Verbindung bezogen auf das absorbierende Hydrogel.

Hydrophobierte Aluminiumoxide und/oder Kieselsäuren lassen sich beispielsweise durch Umsetzung hydrophiler Aluminiumoxide und/oder Kieselsäuren mit Hexamethyldisilazan oder Dimethyldichlorsilan erhalten.

Die hydrophoben und hydrophilen Verbindungen werden vorzugsweise mit dem getrockneten wasserabsorbierenden Hydrogel vermischt. Trocken bedeutet vorzugsweise einen Wassergehalt von weniger als 20 Gew.-%, besonders bevorzugt von weniger als 10 Gew.-%. Die Art des Mischens unterliegt keinen Beschränkungen, vorzugsweise werden Reaktionsmischer oder Misch- und Trocknungsanlagen, wie beispielsweise Lödige®-Mischer, BEPEX®-Mischer, NAUTA®-Mischer, SCHUGGI®-Mischer, NA-RA®-Trockner und PROCESSALL®, verwendet. Überdies können auch Wirbelschichttrockner eingesetzt werden. Die Mischung wird zweckmäßigerweise mit einer Verweilzeit von 1 bis 180 Minuten, vorzugsweise von 2 bis 20 Minuten, besonders bevorzugt von 5 bis 20 Minuten, und einer Drehzahl von 10 bis 1000 U/min, vorzugsweise von 50 bis 300 U/min, besonders bevorzugt von 50 bis 250 U/min, durchgeführt.

Ein weiterer Gegenstand der Erfindung ist ein Verfahren zur Nachbehandlung von absorbierenden Hydrogelen umfassend die Schritte:

- Nachbehandlung mit wenigstens einem mehrwertigen Metallionen, Lösungen mehrwertiger Metallionen, wasserlöslichen kationischen Polymeren und/oder Lösungen von wasserlöslichen Polymeren, vorzugsweise mehrwertigen Metallionen, wie beispielsweise $Al^{3+}$, $Fe^{2+}$, $Fe^{3+}$, $Ti^{3}$, $Ti^{4+}$, $Co^{2+}$, $Ni^{2+}$, $Cr^{3+}$, $Mn^{2+}$, $Zn^{2+}$, $Mg^{2+}$, $Ca^{2+}$, $Zr^{3+}$, $Zr^{4+}$, besonders bevorzugt $Al^{3+}$.

- Nachbehandlung mit wenigstens einer hydrophoben Verbindung, vorzugsweise hydrophobe und/oder hydrophobierte Tonmineralien, hydrophobierte Aluminiumoxide und/oder hydrophobierte Kieselsäuren, besonders bevorzugt hydrophobierte Aluminiumoxide und/oder hydrophobierte Kieselsäuren, wie beispielsweise Aerosil® R 812, Aerosil® R 974 oder Aerosil® R 8200 (Degussa Aktiengesellschaft, DE).

- gegebenenfalls Nachbehandlung mit wenigstens einer hydrophilen Verbindung, vorzugsweise hydrophilen Tonmineralien, Aluminiumoxide und/oder Kieselsäuren, besonders bevorzugt Aluminiumoxide und/oder Kieselsäuren, wie beispielsweise Aerosil® 200 (Degussa Aktiengesellschaft, DE).

- gegebenenfalls Nachbehandlung mit wenigstens einem anionischen, kationischen und/oder nichtionischen Tensid, vorzugsweise einem nichtionischen Tensid, wie beispielsweise Sorbitanester mit einem HLB-Wert von 2 bis 18, besonders bevorzugt ist Span® 80 (Uniqema, NL).

Die Gegenionen der mehrwertigen Metallionen unterliegen keiner Beschränkung, bei Verwendung eines Lösungsmittels sind Gegenionen bevorzugt, die eine ausreichende Löslichkeit gewährleisten, bevorzugt ist Sulfat. Vorzugsweise werden die Metallionen als Lösung dosiert. Besonders bevorzugt als Lösungsmittel ist Wasser. Die Konzentration des mehrwertigen Metallions in der wässrigen Lösung beträgt typischerweise von 1 bis 20 Gew.-%, vorzugsweise 2 bis 10 Gew.-%.

Eingesetzt werden typischerweise 0,05 bis 4 Gew.-%, vorzugsweise 0,1 bis 2 Gew.-%, besonders bevorzugt 0,2 bis 1 Gew.-%, des mehrwertigen Metallions bezogen auf das absorbierede Hydrogel.

Vorzugsweise werden die Tenside als Lösung dosiert. Besonders bevorzugt als Lösungsmittel ist Diethylenglykolmonobutylether. Die Konzentration des Tensids in der Lösung beträgt typischerweise von 5 bis 70 Gew.-%, vorzugsweise 10 bis 50 Gew.-%, besonders bevorzugt 20 bis 30 Gew.-%.

Eingesetzt werden typischerweise 0,01 bis 4 Gew.-%, vorzugsweise 0,05 bis 2 Gew.-%, besonders bevorzugt 0,1 bis 1 Gew.-%, des Tensids bezogen auf das absorbierende Hydrogel.

Die Reihenfolge, in der die Nachbehandlungsmittel dosiert werden unterliegt keiner Beschränkung, bevorzugt ist die Reihenfolge

- mehrwertige Metallionen, Lösungen mehrwertiger Metallionen, wasserlösliche kationische Polymere und/oder Lösungen von wasserlöslichen Polymeren,

- hydrophile und hydrophobe Verbindungen gemeinsam oder getrennt in beliebiger Reihenfolge

- oberflächenaktive Verbindungen, wie beispielsweise Tenside, sowie deren Lösungen,

wobei vorzugsweise nur mehrwertige Metallionen und hydrophobe Verbindungen zur Nachbehandlung eingesetzt werden.

Die gelösten Nachbehandlungsmittel werden vorzugsweise auf das getrocknete wasserabsorbierenden Hydrogel aufgesprüht und gemischt. Die Art des Mischens unterliegt keinen Beschränkungen, vorzugsweise werden Reaktionsmischer oder Misch-und Trocknungsanlagen, wie beispielsweise Lödige®-Mischer, BEPEX®-Mischer, NAUTA®-Mischer, SCHUGGI®-Mischer, NARA®-Trockner und PROCESSALL®, verwendet. Überdies können auch Wirbelschichttrockner eingesetzt werden. Die Mischung wird zweckmäßigerweise mit einer Verweilzeit von 1 bis 180 Minuten, vorzugsweise von 2 bis 15 Minuten, und einer Drehzahl von 10 bis 1000 U/min, vorzugsweise von 50 bis 300 U/min, besonders bevorzugt von 50 bis 250 U/min, durchgeführt.

Nach dem letzten Schritt kann getrocknet werden. Die Trocknung kann im Mischer selbst erfolgen, durch Beheizung des Mantels oder Einblasen von Warmluft. Ebenso geeignet sind ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen im erfindungsgemäßen Verfahren liegen im Bereich 50 bis 250 °C, vorzugsweise bei 50 bis 200 °C, besonders bevorzugt bei 50 bis 150 °C. Die Verweilzeit bei dieser Temperatur im Reaktionsmischer oder Trockner beträgt zweckmäßigerweise unter 30 Minuten, vorzugweise unter 10 Minuten.

Die Trocknung wird vorzugsweise bei vermindertem Druck, vorzugsweise bei weniger als 500 mbar, besonders bevorzugt bei weniger als 200 mbar, durchgeführt und gegebenefalls durch einen trockenen Gasstrom, vorzugsweise Stickstoff, in einer Menge von 20 bis 1000 l/kgh, vorzugsweise 100 bis 250 l/kgh, unterstützt.

Vorzugsweise werden die absorbierenden Hydrogele in dem erfindungsgemäßen Verfahren zusätzlich mit einer hydrophilen organischen Verbindung nachbehandelt. Die hydrophilen organischen Verbindungen verbessern die Fixierung der partikulären Nachbehandlungsmittel auf dem Superabsorber. Geeignete hydrophile organische Verbindungen sind beispielsweise niedere wasserlösliche Polyole mit einem mittleren Molekulargewicht von 100 bis 6.000 g/mol, vorzugsweise 200 bis 3.000 g/mol, besonders bevorzugt von 250 bis 1.000 g/mol.

Bevorzugte hydrophile organische Verbindungen sind dendritische Polymere, hochverzweigte Polymere wie beispielsweise Polyglycerine, Polyethylenglykole, Polypropylenglykole, Statistische oder Block-Copolymere von Ethylenoxid und Propylenoxid. Weitere zu diesem Zweck geeignete Verbindungen sind die Polyethoxylate oder Polypropoxylate von Polyhydroxyverbindungen, wie Glycerin, Sorbitol, Trimethylolpropan, Trimethylolethan, Pentaerythrit. Beispiele hierfür sind n-fach ethxoxyliertes Trimethylolpropan oder Glyzerin wobei n eine ganze Zahl zwischen 1 und 100 darstellt. Weitere Beispiele sind Blockcopolymere wie insgesamt n-fach ethoxyliertes und dann m-fach propoxyliertes Trimethylolpropan oder Glyzerin wobei n eine ganze Zahl zwischen 1 und 40 darstellt und m eine ganze Zahl zwischen 1 und 40 darstellt. Die Reihenfolge der Blöcke kann auch umgekehrt sein.

Die hydrophile organische Verbindung kann vor, während oder nach jedem der Nachbehandlungsschritte zugesetzt werden, vorzugsweise vor der Nachbehandlung mit der hydrophoben organischen Verbindung, besonders bevorzugt zusammen mit dem mehrwertigen Metallion.

Die hydrophile organische Verbindung ist bei 23°C flüssig und weist bei 23°C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, auf.

Die hydrophile organische Verbindung wird üblicherweise in einer Menge von 0,01 bis 2 Gew.-%, vorugsweise von 0,1 bis 1 Gew.-%, besonders bevorzugt von 0,35 bis 0,75 Gew.-%, bezogen auf das getrocknete Hydrogel, eingesetzt.

Ein weiterer Gegenstand der Erfindung sind vernetzte wasserabsorbierende Polymere, die beispielsweise nach dem erfindungsgemäßen Verfahren erhältlich sind, dies sind absorbierende Hydrogele mit einer Blutabsorption von mindestens 10 g/g, vorzugsweise von mindestens 15 g/g, besonders bevorzugt von mindestens 19 g/g, ganz besonders bevorzugt von mindestens 22 g/g und insbesondere bevorzugt von mindestens 25 g/g, und einer Verfestigungszeit von weniger als 120 Sekunden, vorzugsweise weniger als 100 Sekunden, besonders bevorzugt von weniger als 90 Sekunden, ganz besonders bevorzugt von weniger als 80 Sekunden und insbesondere bevorzugt von weniger als 70 Sekunden, sowie einem Schwimmverhalten, bei dem von 40 bis 90 %, besonders bevorzugt von 65 bis 80 %, der zu verdickenden Blut und/oder Körperflüssigkeiten enthaltenden Lösung beginnend von der Flüssigkeitsoberfläche eingedickt wird, sowie deren Anwendung zur Absorption von Blut und/oder Körperflüssigkeiten, insbesondere in Hygieneartikeln, oder zum Verdicken wässriger Lösungen und/oder Suspensionen, insbesondere zum Verdicken medizinischer Abfälle.

Ein weiterer Gegenstand der Erfindung sind Hygieneartikel, welche die erfindungsgemäßen Superabsorber enthalten.

Die im erfindungsgemäßen Verfahren einsetzbaren quellbaren hydrogelbildenden Polymere sind insbesondere Polymere aus vernetzten (co)polymerisierten hydrophilen Monomeren, Polyasparaginsäure, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Bevorzugt handelt es sich bei dem zu vernetzenden Polymer um ein Polymer, das Struktureinheiten enthält, die sich von Acrylsäure oder deren Estern ableiten, oder die durch Pfropfcopolymerisation von Acrylsäure oder Acrylsäureestern auf eine wasserlösliche Polymermatrix erhalten wurden. Diese Hydrogele sind dem Fachmann bekannt und beispielsweise in der US-4,286,082, DE-C-27 06 135, US-A-4,340,706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780,

EP-A-0 205 674, US-A-5,145,906, EP-A-0 530 438, EP-A-0 670 073, US-A-4,057,521, US-A-4,062,817, US-A-4,525,527, US-A-4,295,987, US-A-5,011,892, US-A-4,076,663 oder US-A-4,931,497 beschrieben.

Zur Herstellung dieser quellbaren hydrogelbildenden Polymere geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder auch Diallyldimethyl-ammoniumchlorid. Bevorzugte hydrophile Monomere sind Verbindungen der allgemeinen Formel I

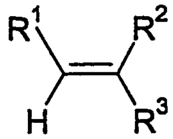

(I)

worin

$R^1$ Wasserstoff, Methyl, Ethyl oder Carboxyl,

$R^2$ $-COOR^4$, Hydroxysulfonyl oder Phosphonyl, eine mit einem $C_1$-$C_4$-Alkanol veresterte Phosphonylgruppe oder eine Gruppe der Formel II

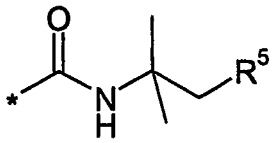

(II)

$R^3$ Wasserstoff, Methyl oder Ethyl,

$R^4$ Wasserstoff, $C_1$-$C_4$-Aminoalkyl, $C_1$-$C_4$-Hydroxyalkyl, Alkalimetall- oder Ammoniumion und

$R^5$ eine Sulfonylgruppe, eine Phosphonylgruppe oder eine Carboxylgruppe oder jeweils deren Alkalimetall- oder Ammoniumsalze, bedeuten.

Beispiele für $C_1$-$C_4$-Alkanole sind Methanol, Ethanol, n-Propanol, Isopropanol oder n-Butanol.

Besonders bevorzugte hydrophile Monomere sind Acrylsäure und Methacrylsäure, sowie deren Alkalimetalloder Ammoniumsalze, beispielsweise Natriumacrylat, Kaliumacrylat oder Ammoniumacrylat.

Geeignete Pfropfgrundlagen für hydrophile Hydrogele, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel III

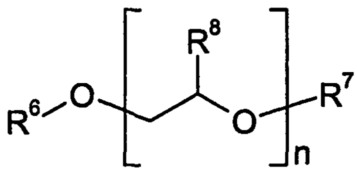

(III)

worin

$R^6$, $R^7$ unabhängig voneinander Wasserstoff, Alkyl, Alkenyl oder Aryl,

$R^8$ Wasserstoff oder Methyl und

n eine ganze Zahl von 1 bis 10000 bedeuten.

$R^6$ und $R^7$ bedeuten bevorzugt Wasserstoff, $C_1$-$C_4$-Alkyl, $C_2$-$C_6$-Alkenyl oder Phenyl.

Bevorzugte Hydrogele sind insbesondere Polyacrylate, Polymethacrylate sowie die in der US-4,931,497, US-5,011,892 und US-5,041,496 beschriebenen Pfropfpolymere.

Die quellbaren hydrogelbildenden Polymere sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin einsetzbar im erfindungsgemäßen Verfahren sind auch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Ethylenglykoldiallylether, Glyceroldi- und Triallylether, Polyallylether auf Basis Sorbitol, sowie ethoxilierte Varianten davon.

Die bevorzugten Herstellverfahren für das im erfindungsgemäßen Verfahren einsetzbare Basispolymer werden in "Modern Superabsorbent Polymer Technology", F.L. Buchholz and A.T. Graham, Wiley-VCH, 1998, Seiten 77 bis 84 beschrieben. Besonders bevorzugt sind Bassispolymere, die im Kneter, wie beispielsweise in WO-A-01/38402 beschrieben, oder auf einem Bandreaktor, wie beispielsweise in EP-A-0 955 086 beschrieben, hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere in Mengen von 0,001 bis 10 mol-%, vorzugsweise 0,01 bis 1 mol-% enthalten, ganz besonders bevorzugt sind jedoch Polymere, die durch radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie beispielsweise Pentaerythritoltriallylether oder Trimethylolpropandiallylether).

Die quellbaren hydrogelbildenden Polymere können durch an sich bekannte Polymerisationsverfahren hergestellt werden. Bevorzugt ist die Polymerisation in wässriger Lösung nach dem Verfahren der sogenannten Gelpolymerisation. Dabei werden beispielsweise 15 bis 50 gew.-%ige wässrige Lösungen eines oder mehrerer hydrophiler Monomere und gegebenenfalls einer geeigneten Pfropfgrundlage in Gegenwart eines Radikalinitiators, bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxoverbindungen wie $(NH_4)_2S_2O_8$ oder $K_2S_2O_8$ oder $H_2O_2$. Sie können gegebenenfalls in Kombination mit Reduktionsmitteln wie Natriumhydrogensulfit und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie Mannichaddukte aus Sulfinsäuren, Aldehyden und Aminoverbindungen, wie sie in der DE-A-13 01 566 beschrieben sind, verwendet werden. Durch mehrstündiges Nachheizen der Polymergele im Temperaturbereich 50 bis 130°C, vorzugsweise 70 bis 100°C, können die Qualitätseigenschaften der Polymere noch verbessert werden.

Die erhaltenen Gele werden zu 50 bis 85 mol-%, bezogen auf eingesetztes Monomer neutralisiert, wobei die üblichen Neutralisationsmittel verwendet werden können, bevorzugt Alkalimetallhydroxide oder -oxide, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt. Das getrocknete Hydrogel wird hiernach gemahlen und gesiebt, wobei zur Mahlung üblicherweise Walzenstühle, Stiftmühlen oder Schwingmühlen eingesetzt werden können. Die Partikelgröße des gesiebten Hydrogels liegt vorzugsweise im Bereich 45 bis 1000 $\mu$m, besonders bevorzugt bei 45-850 $\mu$m, ganz besonders bevorzugt bei 100 bis 800 $\mu$m und noch mehr bevorzugt bei 100 bis 700 $\mu$m.

Die Nachvernetzung von Hydrogelen und Superabsorber wird üblicherweise so durchgeführt, dass eine Lösung

des Oberflächennachvernetzers auf das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Bevorzugt ist das Aufsprühen einer Lösung des Vernetzers in Reaktionsmischern oder Misch- und Trocknungsanlagen, wie beispielsweise Lödige®-Mischer, BEPEX®-Mischer, NAUTA®-Mischer, SCHUGGI®-Mischer, NARA®-Trockner und PROCESSALL®. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250 °C, bevorzugt bei 50 bis 200 °C, und besonders bevorzugt bei 50 bis 150 °C. Die bevorzugte Verweilzeit bei dieser Temperatur im Reaktionsmischer oder Trockner beträgt unter 30 Minuten, besonders bevorzugt unter 10 Minuten.

Der Vernetzer wird bevorzugt in nicht selbst-reaktiven Lösemitteln gelöst, bevorzugt in niederen Alkoholen, wie beispielsweise Methanol, Ethanol, Propandiol, Ethylenglykol, ganz besonders bevorzugt in wässrigen Lösungen solcher geeigneter Alkohole, wobei der Alkoholgehalt der Lösung 10 bis 90 Gew.-%, besonders bevorzugt 40 bis 60 Gew.-% beträgt.

Der Vernetzer wird dabei in einer Menge von 0,01 bis 1 Gew.-%, bezogen auf das eingesetzte Polymer, verwendet, und die Vernetzerlösung selbst in einer Menge von 1 bis 20 Gew.-%, bevorzugt 5 bis 15 Gew.-%, bezogen auf das eingesetzte Polymer, verwendet.

Der AUL-0,7psi-Wert [g/g] der erfindungsgemäßen nachvemetzten wasserabsorbierenden Polymere kann nach der in DE-A-199 09 653 angegebenen Methode gemessen werden und ist bevorzugt größer 10, insbesondere größer 15, besonders bevorzugt größer 20, insbesondere größer 25, insbesondere bevorzugt größer 30.

Die erfindungsgemäßen nachbehandelten wässrige Flüssigkeiten absorbierenden Hydrogele eignen sich besonders zum Verdicken wässriger Lösungen und/oder Suspensionen, vorzugsweise zum Verdicken wäßriger Abfalllösungen und/oder Abfallsuspensionen, wie beispielsweise medizinischer und/oder radioaktiver Abfälle, ganz besonders bevorzugt zum Verdicken medizinischer Abfallösungen und/oder sonders bevorzugt zum Verdicken medizinischer Abfallösungen und/oder Abfallsuspensionen.

Die erfindungsgemäßen nachbehandelten wässrige Flüssigkeiten absorbierenden Hydrogele eignen sich aber auch zur Absorption von Blut und/oder Körperflüssigkeiten in Hygieneartikeln, wie beispielsweise Inkontinenzartikeln, Binden, Tampons, Einlagen. Dazu können die erfindungsgemäßen nachbehandelten wässrige Flüssigkeiten absorbierenden Hydrogele mit Fasern, wie beispielsweise Zellulose, sowie Faservlies zu absorbierenden Verbundstoffen verarbeitet werden.

Durch eine Nachbehandlung mit hydrophober und hydrophiler Materialien wird bei der Behandlung zumindest teilweise flüssiger Abfälle, inbesonderere zumindest teilweise flüssiger medizinischer Abfälle, eine gleichmäßige Verteilung des Superabsorbers im Behälter erreicht, wobei wenigstens ein Teil des Superabsorbers zu Beginn auf der Oberfläche der Lösung schwimmt.

Erfindungsgemäß sind ebenfalls Mischungen von Produkten hergestellt nach dem erfindungsgemäßen Verfahren mit bioziden, antimikrobiellen und/oder antibakteriellen Stoffen und/oder Parfüm- oder Duftstoffen, Stabilisatoren, Farbstoffen, pH-Indikatoren und/oder anderen Hilfsmitteln.

Zur Bestimmung der Güte der erfindungsgemäßen Nachbehandlung wird das getrocknete Hydrogel mit den Testmethoden geprüft, die nachfolgend beschrieben sind:

Methoden:

Blutabsorption (BA):

Diese Methode dient zur Bestimmung der Blutabsorption von Superabsorbern innerhalb von 30 Minuten.

Versuchsaufbau:

- Kunststoffbehälter, rund, Innendurchmesser 50 ± 0,2 mm, Innenhöhe 20 ± 0,2 mm (Behälter I)
- Kunststoffzylinder mit Netzteil (400 mesh = 36 μm große Löcher), Innendurchmesser 25 ± 0,2 mm, Höhe 40 ± 0,2 mm (Behälter II)
- Petrischale mit Deckel, Durchmesser 140 mm, Höhe 75 mm
- Stoppuhr
- Analysenwaage mit einer Genauigkeit von ± 0,0001g.
- Defibriniertes Schafsblut der Firma Oxoid GmbH, D-46467 Wesel

Versuchsdurchführung:

0,2 g Superabsorber werden in den Behälter II, dessen Leergewicht vorher bestimmt wurde, eingewogen. Behälter I wird mit 15 g defibriniertem Schafsblut beschickt. Behälter II wird nun in Behälter I gestellt, dieser Aufbau in die Petri-Schale gestellt, die Petri-Schale durch den Deckel verschlossen und die Zeitmessung gestartet. Nach einer Zeit von 30 Minuten wird Behälter II aus Behälter I genommen, die äußere Seite des Behälters II mit einem Tuch gereinigt und das Gewicht des Behälters II anschließend bestimmt. Aus der Differenz dieses Gewichtes und des Leergewichtes von Behälter II sowie der eingesetzten Superabsorber-Masse (0,2 g) wird die absorbierte Blutmenge und daraus die Blutabsorption berechnet.

Berechnung:

$$\frac{\text{Absorption an Blut [g]}}{\text{Einwaage Superabsorber [g]}} = \text{Blutabsorption [g/g]}$$

Verfestigungszeit (Solidification Time ST):

Diese Methode dient als Labormethode zur Bestimmung der Zeit, die ein Superabsorber braucht, um 2 Liter einer 0,9 gew.-%igen NaCl-Lösung zu verfestigen und stellt somit den spezifikationsgerechten Labortest der Verdickung von Krankenhaus- oder medizinischen Abfällen dar.

Versuchsaufbau:

- Zylindrischer Glasbehälter mit einem Innendurchmesser von 12 cm, einer Höhe von 28 cm und einem Innenvolumen von 3 Litern
- 2 Liter 0,9 gew.-%ige NaCl-Lösung, die durch Auflösen von 90 g NaCl in 9910 ml vollentsalztem Wasser hergestellt wurden
- Stoppuhr

Versuchsdurchführung:

Der Glasbehälter wird mit 2 Litern der 0,9 gew.-%iger NaCl-Lösung beschickt. Anschließend werden 67 g Superabsorber auf einmal zur Lösung gegeben und die Zeit bis zur vollständigen Verfestigung der Lösung gemessen.

Schwimmverhalten (SV):

Das Schwimmverhalten des zu untersuchenden Superabsorbers wird zusammen mit der Verfestigungszeit bestimmt. Während der Messung wird dabei eine visuelle Bestimmung vorgenommen.

Superabsorberpartikel, die auf der Flüssigkeitsoberfläche schwimmen, quellen ausgehend von der Flüssigkeitsoberfläche in Richtung Behälterboden. Superabsorberpartikel, die zu Boden sinken, quellen ausgehend vom Behälterboden in Richtung Flüssigkeitsoberfläche.

Ein Schwimmverhalten von 0% ist so definiert, dass alle Superabsorberpartikel sofort zu Boden sinken und die zu verdickende Flüssigkeit ausschließlich beginnend vom Behälterboden verdickt wird. Die Quellfront wandert dabei vom Behälterboden bis zur Flüssigkeitsoberfläche.

Ein Schwimmverhalten von 100% ist so definiert, dass alle Superabsorberpartikel auf der Flüssigkeitsoberfläche schwimmen und die zu verdickende Flüssigkeit ausschließlich beginnend von der Flüssigkeitsoberfläche verdickt wird. Die Quellfront wandert dabei von der Flüssigkeitsoberfläche bis zum Behälterboden.

Ein Schwimmverhalten von 50% ist so definiert, dass die Superabsorberpartikel teilweise sofort zu Boden sinken und teilweise auf der Flüssigkeitsoberfläche schwimmen, so dass sich die beiden Quellfronten in der Mitte zwischen Behälterboden und Flüssigkeitsoberfläche treffen.

Beispiele

Beispiele 1 bis 31:

In einem Lödige®-Pflugscharmischer vom Typ M5/20 Laborchargenmischer wurden 1 kg kommerziell verfügbares wässrige Flüssigkeiten absorbierendes Hydrogel (Hysorb F) vorgelegt und

ggf. die angegebene Menge an Aluminiumsulfat als 26,8 gew.-%ige wässrige Lösung aufgesprüht und 10 Minuten nachgemischt und

ggf. die angegebene Menge an Aerosil® R 812, Aerosil® R 974, Aerosil® R 8200 und/oder Aerosil® 200 zugesetzt und 15 Minuten nachgemischt und

ggf. die angegebene Menge an Span® 80 als 25 gew.-%ige Lösung in Diethylenglykolmonobutylether aufgesprüht und 10 Minuten nachgemischt.

Die Drehzahl des Mischers betrug 125 U/min.

In den Beispielen, in denen die wässrige Aluminiumsulfatlösung aufgesprüht wurde, wurde zusätzlich getrocknet. Getrocknet wurde bei 70°C, einem Druck von 150 mbar und einer Verweilzeit von 16 Stunden. Die Trocknung wurde durch eine Gasstrom von 200 l/h Stickstoff unterstützt.

Die Ergebnisse sind in der nachfolgenden Tabelle zusammengestellt.

Tabelle 1: Beispiele auf Basis Hysorb® F

| Beispiel | Additiv 1 | Additiv 2 | Additiv 3 | Additiv 4 | BA [g/g] | ST [sek] | SV [%] |
|---|---|---|---|---|---|---|---|
| 1 | | | | | 16,5 | >600 | 0 |
| 2 | 0,06% Aerosil R 8200 | | 4% $Al_2(SO_4)_3$ | | | 77 | 65 |
| 3 | 0,17% Aerosil R 8200 | | 4% $Al_2(SO_4)_3$ | | 7,5 | 93 | 60 |
| 4 | 0,25% Aerosil R 812 | 1% Aerosil 200 | | | 19,8 | 300 | 20 |
| 5 | 0,5% Aerosil R 974 | 2% Aerosil 200 | | | 16,1 | 265 | 25 |
| 6 | | | 1% $Al_2(SO_4)_3$ | | 18,9 | >1500 | 0 |
| 7 | | | 5% $Al_2(SO_4)_3$ | | 21,0 | 270 | 0 |
| 8 | | | 10% $Al_2(SO_4)_3$ | | 22,5 | 174 | 0 |
| 9 | 0,5% Aerosil R 974 | | | | 12,3 | >600 | 100 |
| 10 | 2% Aerosil R 974 | | | | 5,6 | >600 | 100 |
| 11 | | | | 0,1% Span 80 | 13,4 | >600 | 0 |
| 12 | | | | 0,25% Span 80 | 17,7 | >600 | 0 |
| 13 | | | | 0,5% Span 80 | 18,4 | 2100 | 0 |
| 14 | | | | 1% Span 80 | 16,3 | >1200 | 0 |
| 15 | | 0,1% Aerosil 200 | 4% $Al_2(SO_4)_3$ | | 18,9 | 120 | 0 |

(fortgesetzt)

| Beispiel | Additiv 1 | Additiv 2 | Additiv 3 | Additiv 4 | BA [g/g] | ST [sek] | SV [%] |
|---|---|---|---|---|---|---|---|
| 16 | | 0,3% Aerosil 200 | 4% $Al_2(SO_4)_3$ | | 19,5 | 138 | 0 |
| 17 | | 0,5% Aerosil 200 | 4% $Al_2(SO_4)_3$ | | 19,7 | 120 | 0 |
| 18 | | 1% Aerosil 200 | 4% $Al_2(SO_4)_3$ | | 20,2 | >600 | 0 |
| 19 | | 0,5% Aerosil 200 | 4% $Al_2(SO_4)_3$ | 0,5% Span 80 | 15 | 150 | 20 |
| 20 | | | 1% $Al_2(SO_4)_3$ | 0,25% Span 80 | 17,6 | 264 | 0 |
| 21 | | | 4% $Al_2(SO_4)_3$ | 1% Span 80 | 20,9 | 189 | 0 |
| 22 | | 0,5% Aerosil 200 | | 0,25% Span 80 | 18,4 | >600 | 100 |
| 23 | | 1% Aerosil 200 | | 0,1% Span 80 | 16,7 | >600 | 100 |
| 24 | 0,5% Aerosil R 974 | | 1% $Al_2(SO_4)_3$ | | 20,2 | 280 | 100 |
| 25 | 1% Aerosil R 974 | | 4% $Al_2(SO_4)_3$ | | 17,4 | 414 | 100 |
| 26 | 0,5% Aerosil R 974 | 0,5% Aerosil 200 | 4% $Al_2(SO_4)_3$ | 0,25% Span 80 | 19,7 | 66 | 70 |
| 27 | 0,75% Aerosil R 974 | 1% Aerosil 200 | 4% $Al_2(SO_4)_3$ | 0,25% Span 80 | 28,2 | 60 | 70 |
| 28 | 0,25% Aerosil R 974 | 0,5% Aerosil 200 | 4% $Al_2(SO_4)_3$ | 0,25% Span 80 | 22,8 | 60 | 80 |
| 29 | 0,5% Aerosil R 974 | 0,5% Aerosil 200 | 4% $Al_2(SO_4)_3$ | 0,25% Span 80 | 27,7 | 48 | 80 |
| 30 | | 1% Aerosil 200 | | | 16,9 | >600 | 0 |
| 31 | | 2% Aerosil 200 | | | 17,7 | >600 | 0 |

Hysorb® F: superabsorbierendes Hydrogel (BASF Aktiengesellschaft, DE)
Aerosil® R 812: hydrophobierte pyrogene Kieselsäure (Degussa, DE)
Aerosil® R 974: hydrophobierte pyrogene Kieselsäure (Degussa, DE)
Aerosil® R 8200: hydrophobierte pyrogene Kieselsäure (Degussa, DE)
Aerosil® 200: hydrophile pyrogene Kieselsäure (Degussa, DE)
Span® 80: Sorbitanmonooleat (Uniqema, NL)

Das Besprühen mit Aluminiumsulfatlösung (Beispiele 6 bis 8) führt zu einer geringfügig verbesserten Blutabsorption und zu einer etwas verkürzten Verfestigungszeit. Die dazu notwendigen Mengen an Aluminiumsulfat sind hoch. Das Schwimmverhalten der nur mit Aluminiumsulfat behandelten Hydrogele ist ungenügend.

Das Abmischen mit hydophobierter pyrogener Kieselsäure (Beispiele 9 und 10) führt zwar zu schwimmfähigen Hydrogelen, die Blutabsorption ist aber drastisch verschlechtert.

Das Besprühen mit Span® 80 (Beispiele 11 bis 14) führt zu keinen verbesserten Produkteigenschaften. Dies gilt ebenso für das Abmischen mit pyrogener Kieselsäure (Beispiele 30 und 31).

Damit führt keine der Nachbehandlungen für sich allein zu Produkten mit den erfindungsgemäßen Eigenschaften.

Durch die Nachbehandlung des getrockneten Hydrogels mit geringen Mengen an hydrophobierter Kieselsäure wurden Hydrogele mit hervorragendem Schwimmverhalten erhalten. In Beispiel 3 wurden zusätzlich 0,35 Gew.-% Polyethylenglykol mit einem mittleren Molekulargewicht von 300 g/mol zugesetzt um die Bindung der Kieselsäurepartikel auf dem Hydrogel zu verbessern.

Das Abmischen mit pyrogener Kieselsäure und hydophobierter pyrogener Kieselsäure (Beispiele 4 und 5) führt zu Hydrogelen mit hervorragendem Schwimmverhalten.

Die Verwendung pyrogener Kieselsäure zusammen mit Aluminiumsulfatlösung (Beispiele 15 bis 18) führt zu einer gesteigerten Blutabsorption und einer verkürzten Verfestigungszeit. Die nachbehandelten Hydrogele sind aber nicht schwimmfähig.

Die Verwendung von Span® 80 zusammen mit Aluminiumsulfatlösung (Beispiele 20 und 21) führt ebenfalls zu einer gesteigerten Blutabsorption und einer verkürzten Verfestigungszeit. Die nachbehandelten Hydrogele sind aber auch nicht schwimmfähig.

Die Verwendung von Span® 80 zusammen mit pyrogener Kieselsäure (Beispiele 22 und 23) liefert schwimmfähige Hydrogele. Nachteilig ist, dass keine Hydrogelanteile absinken und dass die Verfestigungszeit unverändert lang ist.

Ebenso führt die Verwendung von Aluminiumsulfatlösung und hydrophobierter pyrogener Kieselsäure (Beispiele 24 und 25) zu schwimmfähigen Hydrogelen. Auch hier ist aber nachteilig, dass keine Hydrogelanteile absinken. Die Blutabsorption ist geringfügig erhöht und die Verfestigungszeit ist geringfügig verkürzt.

Die dreistufige Nachbehandlung a) Besprühen mit Aluminiumsulfatlösung, b) Abmischen mit pyrogener Kieselsäure und c) Besprühen mit Span® 80-Lösung (Beispiel 19) ergibt ein Hydrogel mit gutem Schwimmverhalten und verkürzter Verfestigungszeit. Die Blutabsorption ist aber gegenüber dem Ausgangsprodukt vermindert.

Ein ganz besonders vorteilhaftes Eigenschaftsprofil wird mit der vierstufigen Nachbehandlung a) Besprühen mit Aluminiumsulfatlösung, Abmischen mit b) pyrogener Kieselsäure und c) hydrophobierter pyrogener Kieselsäure sowie d) Besprühen mit Span® 80-Lösung (Beispiele 26 bis 29) erzielt. Die so nachbehandelten Hydrogele zeigen eine deutlich verbesserte Blutabsorption sowie ein optimales Schwimmverhalten. Das Hydrogel schwimmt teilweise auf der Lösung und das quellende Hydrogel sperrt damit die Flüssigkeitspoberfläche nach oben ab, andererseits sinken Hydrogelanteile ab und führen aufgrund der verbesserten Blutabsorption zu einer sehr kurzen Verfestigungszeit.

## Patentansprüche

1. Hydrogel, wobei das Hydrogel zu 50 bis 85 mol-% neutralisiert und mit einer hydrophoben Verbindung und gegebenenfalls einer hydrophilen Verbindung nachbehandelt wurde, mit einem Schwimmverhalten, bei dem eine 0,9gew.-%ige Kochsalzlösung zu 40 bis 90 % beginnend von der Flüssigkeitsoberfläche und der restliche Teil der 0,9gew.-%ige Kochsalzlösung beginnend vom Behälterboden eingedickt wird, und einer Verfestigungszeit, bei der 2 Liter einer 0,9gew.-%igen Kochsalzlösung mit 67 g Hydrogel verfestigt werden, von weniger als 120 Sekunden und einer Blutabsorption von mindestens 10 g/g.

2. Hydrogel gemäß Anspruch 1 bei dem die Verfestigungszeit weniger als 80 Sekunden und/oder die Blutabsorption mindestens 15 g/g beträgt.

3. Verfahren zur Herstellung eines Hydrogels gemäß einem der Ansprüche 1 oder 2, **dadurch gekennzeichnet, dass** ein getrocknetes Hydrogel mit einer hydrophoben Verbindung und gegebenenfalls einer hydrophilen Verbindung nachbehandelt wird.

4. Verfahren gemäß Anspruch 3, **dadurch gekennzeichnet, dass** es sich bei den hydrophoben und gegebenenfalls hydrophilen Verbindungen um Partikel mit einem mittleren Durchmesser von 0,001 bis 10 $\mu$m handelt.

5. Verfahren gemäß Anspruch 3 oder 4, **dadurch gekennzeichnet, dass** es sich bei den hydrophoben Verbindungen um hydrophobierte Kieselsäuren oder hydrophobierte Gemische aus Kieselsäuren und Aluminiumoxiden handelt.

6. Verfahren gemäß einem der Ansprüche 3 bis 5, **dadurch gekennzeichnet, dass** es sich bei den hydrophilen Verbindungen um Kieselsäuren oder Gemische aus Kieselsäuren und Aluminiumoxiden handelt.

7. Verfahren gemäß einem der Ansprüche 3 bis 6, **dadurch gekennzeichnet, dass** das Hydrogel zusätzlich mit einem mehrwertigen Kation und gegebenenfalls einem Tensid nachbehandelt wird.

8. Verfahren gemäß Anspruch 7, **dadurch gekennzeichnet, dass** es sich bei dem mehrwertigen Kation um ein

Aluminiumion handelt.

9. Verfahren gemäß Anspruch 7 oder 8, **dadurch gekennzeichnet, dass** es sich bei dem Tensid um einen Sorbitanester handelt.

10. Verfahren gemäß einem der Ansprüche 7 bis 9, **dadurch gekennzeichnet, dass** das mehrwertige Kation als wässrige Lösung dosiert wird und das Tensid einen HLB-Wert von weniger als 18 aufweist.

11. Verfahren gemäß einem der Ansprüche 3 bis 10, **dadurch gekennzeichnet, dass** das nachbehandelte Hydrogel getrocknet wird.

12. Verwendung der Hydrogele gemäß Anspruch 1 oder 2 zur Absorption von Blut und/oder Köperflüssigkeiten oder zum Verdicken wässriger Lösungen und/oder Suspensionen.

13. Verwendung der Hydrogele gemäß Anspruch 1 oder 2 zum Verdicken medizinischer Abfälle.

14. Verwendung gemäß Anspruch 12 in Hygieneartikeln.

15. Hygieneartikel enthaltend ein Hydrogel gemäß einem der Anspruch 1 oder 2.

16. Mischungen von Produkten hergestellt nach einem der Ansprüche 3 bis 11, mit bioziden, antimikrobiellen und/oder antibakteriellen Stoffen und/oder Parfüm- oder Duftstoffen, Stabilisatoren, Farbstoffen, pH-Indikatoren und/oder anderen Hilfsmitteln.

## Claims

1. A hydrogel 50-85 mol% neutralized, aftertreated with a hydrophobic compound and optionally a hydrophilic compound and having a floatability where from 40% to 90% of a 0.9% by weight sodium chloride solution thickens from the surface of the liquid and the rest of the 0.9% by weight sodium chloride solution from the bottom of the container, and a solidification time of less than 120 seconds for solidifying 2 litters of a 0.9% by weight sodium chloride solution with 67 g of hydrogel, and a blood absorbence of at least 10 g/g.

2. The hydrogel according to claim 1 where the solidification time is less than 80 seconds and/or the blood absorbence is at least 15 g/g.

3. A process for preparing a hydrogel according to either of claims 1 and 2, which comprises aftertreating a dried hydrogel with a hydrophobic compound and optionally a hydrophilic compound.

4. The process according to claim 3 wherein the hydrophobic and optionally hydrophilic compounds are particles having an average diameter from 0.001 to 10 μm.

5. The process according to claim 3 or 4 wherein the hydrophobic compounds are hydrophobicized silicas or hydrophobicized mixtures of silicas and aluminas.

6. The process according to any one of claims 3 to 5 wherein the hydrophilic compounds are silicas or mixtures of silicas and aluminas.

7. The process according to any one of claims 3 to 6 wherein the hydrogel is additionally aftertreated with a multivalent cation and optionally a surfactant.

8. The process according to claim 7 wherein the multivalent cation is an aluminum ion.

9. The process according to claim 7 or 8 wherein the surfactant is a sorbitan ester.

10. The process according to any one of claims 7 to 9 wherein the multivalent cation is metered as an aqueous solution and the surfactant has an HLB value of less than 18.

11. The process according to any one of claims 3 to 10 wherein the aftertreated hydrogel is dried.

12. The use of the hydrogels according to claim 1 or 2 for absorbing blood and/or body fluids or for thickening aqueous solutions and/or suspensions.

13. The use of the hydrogels according to claim 1 or 2 for thickening medical wastes.

14. The use according to claim 12 in hygiene articles.

15. Hygiene articles comprising a hydrogel according to either of claims 1 and 2.

16. Mixtures of products prepared according to any one of claims 3 to 11 with biocidal, antimicrobial and/or antibacterial materials and/or perfuming or scent materials, stabilizers, dyes, pH indicators and/or other auxiliaries.

## Revendications

1. Hydrogel, l'hydrogel ayant été neutralisé à raison de 50 à 85% en mole et post-traité avec un composé hydrophobe et le cas échéant un composé hydrophile, présentant un comportement de flottaison, une solution de chlorure de sodium à 0,9% en poids étant épaissie à raison de 40 à 90% en commençant à la surface du liquide et la partie résiduelle de la solution de chlorure de sodium à 0,9% en poids étant épaissie à partir du fond du récipient, et un temps de solidification, 2 litres d'une solution à 0,9% en poids de chlorure de sodium étant solidifiées par 67 g d'hydrogel en moins de 120 secondes, et une absorption de sang d'au moins 10 g/g.

2. Hydrogel selon la revendication 1, le temps de solidification étant inférieur à 80 secondes et/ou l'absorption de sang étant d'au moins 15 g/g.

3. Procédé pour la préparation d'un hydrogel selon l'une quelconque des revendications 1 ou 2, **caractérisé en ce qu'**un l'hydrogel séché est post-traité par un composé hydrophobe et le cas échéant un composé hydrophile.

4. Procédé selon la revendication 3, **caractérisé en ce qu'**il s'agit, pour les composés hydrophobes et le cas échéant hydrophiles, de particules présentant un diamètre moyen de 0,001 à 10 $\mu$m.

5. Procédé selon la revendication 3 ou 4, **caractérisé en ce qu'**il s'agit, pour les composés hydrophobes, de silices hydrofugées ou de mélanges hydrofugés de silices et d'oxydes d'aluminium.

6. Procédé selon l'une quelconque des revendications 3 à 5, **caractérisé en ce qu'**il s'agit, pour les composés hydrophiles, de silices ou de mélanges de silices et d'oxydes d'aluminium.

7. Procédé selon l'une quelconque des revendications 3 à 6, **caractérisé en ce que** l'hydrogel est en outre post-traité par un cation polyvalent et le cas échéant un agent tensioactif.

8. Procédé selon la revendication 7, **caractérisé en ce qu'**il s'agit, pour le cation polyvalent, d'un ion d'aluminium.

9. Procédé selon la revendication 7 ou 8, **caractérisé en ce qu'**il s'agit, pour l'agent tensioactif, d'un ester de sorbitane.

10. Procédé selon l'une quelconque des revendications 7 à 9, **caractérisé en ce que** le cation polyvalent est dosé sous forme de solution aqueuse et que l'agent tensioactif présente une valeur BHL inférieure à 18.

11. Procédé selon l'une quelconque des revendications 3 à 10, **caractérisé en ce que** l'hydrogel post-traité est séché.

12. Utilisation des hydrogels selon la revendication 1 ou 2 pour l'absorption de sang et/ou de liquides corporels ou pour l'épaississement de solutions et/ou de suspensions aqueuses.

13. Utilisation des hydrogels selon la revendication 1 ou 2 pour l'épaississement de déchets médicaux.

14. Utilisation selon la revendication 12 dans des articles hygiéniques.

**15.** Articles hygiéniques contenant un hydrogel selon l'une quelconque des revendications 1 ou 2.

**16.** Mélanges de produits préparés selon l'une quelconque des revendications 3 à 11, avec des biocides, des substances antimicrobiennes et/ou antibactériennes et/ou des parfums ou des substances odoriférantes, des stabilisateurs, des colorants, des indicateurs de pH et/ou d'autres adjuvants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9955767 A
- DE 19909653 A
- WO 0010496 A
- WO 9517455 A
- EP 0759460 A
- WO 9519191 A
- WO 9842193 A
- CA 2188838 A
- WO 9515771 A
- JP 3044367 A
- JP 6345980 A
- US 4286082 A
- DE 2706135 C
- US 4340706 A
- DE 3713601 C
- DE 2840010 C
- DE 4344548 A
- DE 4020780 A
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 0801483 A
- EP 0455985 A
- EP 0467073 A
- EP 0312952 A
- EP 0205874 A
- EP 0499774 A
- DE 2612846 A
- EP 0205674 A
- US 5145906 A
- EP 0530438 A
- EP 0670073 A
- US 4057521 A
- US 4062817 A
- US 4525527 A
- US 4295987 A
- US 5011892 A
- US 4076663 A
- US 4931497 A
- US 5041496 A
- EP 0343427 A
- WO 0138402 A
- EP 0955086 A
- DE 1301566 A


### In der Beschreibung aufgeführte Nicht-Patentliteratur


- **Colloid.** *Polym. Sci.,* 1981, vol. 259, 391-394
- **F.L. Buchholz ; A.T. Graham.** Modern Superabsorbent Polymer Technology. Wiley-VCH, 1998, 77-84
- *Makromol. Chem.,* 1947, vol. 1, 169